Europäisches Patentamt

European Patent Office

Office européen des brevets

(19)

(11) **EP 1 173 756 B1**

(12) **EUROPEAN PATENT SPECIFICATION**

(45) Date of publication and mention
of the grant of the patent:
**12.03.2003 Bulletin 2003/11**

(51) Int Cl.$^7$: **G01N 33/28**, G01N 27/18

(86) International application number:
**PCT/GB00/01363**

(21) Application number: **00927367.3**

(22) Date of filing: **17.04.2000**

(87) International publication number:
**WO 00/065343 (02.11.2000 Gazette 2000/44)**

(54) **MEASUREMENT OF FLUID CONCENTRATIONS**

VERFARHUNG ZUM BESTIMMUNG FLÜSSIGEN KONZENTRATION

MESURE DE CONCENTRATIONS DE FLUIDE

(84) Designated Contracting States:
**AT BE CH CY DE DK ES FI FR GB GR IE IT LI LU
MC NL PT SE**

(30) Priority: **22.04.1999 GB 9909217**

(43) Date of publication of application:
**23.01.2002 Bulletin 2002/04**

(73) Proprietor: **Lattice Intellectual Property Limited
London SW1Y 4UR (GB)**

(72) Inventors:
 • **THURSTON, Robert Richard
 Melbourne, Derbyshire DE73 1BZ (GB)**

 • **PRICE, Barry Leonard
 Quorn, Leicestershire LE12 8RJ (GB)**

(74) Representative: **Illingworth-Law, William et al
Lattice Intellectual Property Ltd
23 Buckingham Gate
London SW1E 6LB (GB)**

(56) References cited:
**WO-A-00/11465          US-A- 4 804 632
US-A- 4 902 138          US-A- 5 353 765**

 • **PATENT ABSTRACTS OF JAPAN vol. 1996, no.
06, 28 June 1996 (1996-06-28) & JP 08 050109 A
(YAMATAKE HONEYWELL CO LTD), 20
February 1996 (1996-02-20)**

**EP 1 173 756 B1**

## Description

The present invention relates to the measurement of the concentration of one or more constituents of a fluid mixture, particularly liquid natural gas (LNG).

LNG is currently being used as an alternative clean fuel for large vehicles such as buses and trucks. The LNG production process removes the carbon dioxide, water and odourant present in natural gas and leaves essentially only methane, ethane, propane and butane.

Operational problems have been observed with LNG vehicles which are due to the gas composition in a vehicle's tank and fuel line changing with time as shown by the chromatographic analysis of bagged samples from "fresh" and "old" tank gases shown below:-

| % volume | methane ($CH_4$) | ethane ($C_2H_6$) | propane ($C_3H_8$) | butane ($C_4H_{10}$) |
|---|---|---|---|---|
| "Fresh" LNG | | | | |
| sample 1 | 98.24 | 1.51 | 0.20 | 0.05 |
| sample 2 | 99.88 | 0.12 | 0.00 | 0.00 |
| "Old" LNG | | | | |
| sample 1 | 84.76 | 13.15 | 1.63 | 0.40 |
| sample 2 | 83.10 | 14.31 | 2.01 | 0.49 |

The shift in the relative proportions of methane to non-methane components with time can be clearly seen.

This variation in LNG gas composition can affect the performance of the engine and may lead to damage through "knock". It has been proposed that this composition change is caused by preferential "boil off" of methane from the LNG mixture. Methane has a much lower boiling point than the other components as shown below:

| | Methane | Ethane | Propane | Butane |
|---|---|---|---|---|
| Boiling point °C | -161.5 | -88.6 | -42.1 | -0.5 |

US-A-4902138 discloses determining the concentrations of the components of a gas blend by measuring the thermal conductivity of the blend at a number of different temperatures, the number of different temperatures being at least as many as one less than the number of components in the blend

According to a first aspect of the present invention there is provided a method of determining the proportion of one of the components or the ratio of two of the components of a fluid mixture of liquid natural gas, the method comprising measuring the thermal conductivity of the liquid natural gas at a first temperature, measuring the thermal conductivity of the liquid natural gas at a second temperature and inferring the proportion of one of the components or the ratio of two of the components of the liquid natural gas from the two thermal conductivity

According to a second aspect of the present invention there is provided a liquid natural gas one component proportion or two component ratio determining apparatus, the apparatus comprising means to measure the thermal conductivity of the liquid natural gas at two different temperatures and control means to infer the proportion of one of the components or the ratio of two of the components of the liquid natural gas from the two thermal conductivity measurements.

The apparatus is preferably used with an engine management system to ensure appropriate operation of a liquid natural gas fuelled engine despite fluctuations in the proportion of components of liquid natural gas supplied to the engine.

An example of the present invention is described below with reference to the accompanying drawings in which:

Figure 1 diagrammatically shows an apparatus to determine the proportion of at least one component of a fluid mixture of liquid natural gas flowing in a conduit;

Figure 2 is a flow diagram of the procedure followed by a control means in the apparatus of Figure 1;

Figure 3 is a graph showing the determined methane proportion of a number of mixtures plotted against the actual methane proportion of the mixtures;

Figure 4 is a graph showing the determined ethane/methane ratio of a number of mixtures plotted against the actual ethane/methane ratio of the mixtures;

Figure 5 diagrammatically shows a thermal conductivity sensor which may be used in the apparatus to determine the proportion of at least one component of an LNG mixture positioned in the cut away wall of a conduit;

Figure 6 diagrammatically shows an electrical circuit for operating a thermal conductivity sensor;

Figure 7 shows the sequence of operations for controlling a sensor; and

Figure 8 shows a thermal conductivity sensor characteristic.

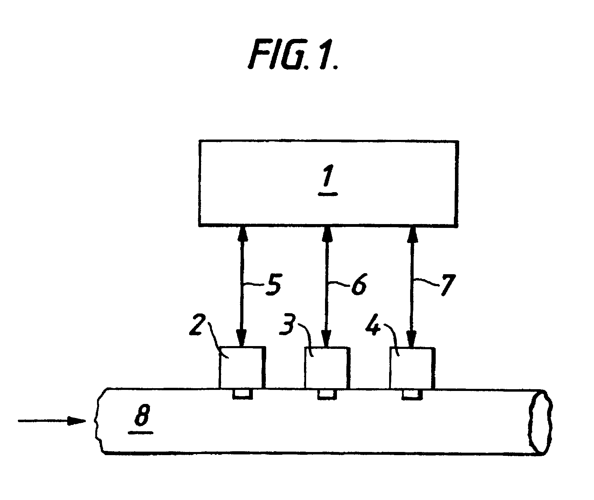

The apparatus shown in Figure 1 comprises a control means 1 which may be a computer or a microprocessor for example connected to a thermal conductivity sensor 2, a temperature sensor 3, and an absolute pressure sensor 4 by connectors 5, 6 and 7 respectively. In this example the thermal conductivity sensor 2, temperature sensor 3 and pressure sensor 4 are mounted to and arranged to take measurements of the contents of a conduit arranged to transport LNG and which may be a fuel line for supplying LNG to an engine. The thermal conductivity sensor and temperature sensor may take any suitable form as are well known in the art. However, in a preferred example the thermal conductivity sensor 2 and temperature sensor 3 are combined in a single unit as described later. The absolute pressure sensor may take any suitable form such as an orifice plate or a by-pass venturi as are well known in the art.

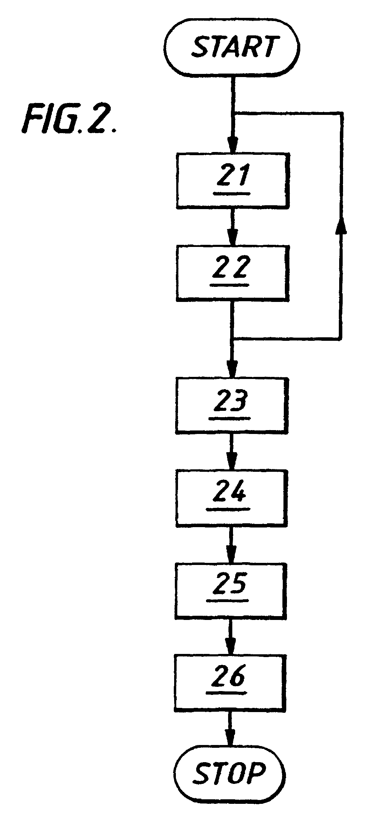

When the proportion of methane or the ethane/methane ratio of the contents of the conduit 8 is to be determined the control means 1 follows the procedure shown in Figure 2. At step 21 the control means 1 instructs the thermal conductivity sensor 2 to take a thermal conductivity measurement of the contents of the conduit 8 at a first temperature and stores the result, ThC(St). At step 22 the control means 1 instructs the thermal conductivity sensor 2 to take a thermal conductivity measurement of the contents of the conduit 8 at a second temperature and stores the result ThC (R). For more precise results steps 21 and 22 may be repeated one or more times to obtain an average value for the thermal conductivity at each of the first and second temperatures. In the preferred example 5000 results are taken with a 1 second delay between each reading to provide time for the detector to settle to the measuring temperature.

At step 23 the control means 1 reads the ambient temperature T of the contents of the conduit 8 from the temperature sensor 3 and stores the result and at step 24 the control means 1 reads the absolute pressure P of the contents of the conduit 8 and stores the result.

At step 25, to determine the proportion of methane in the LNG mixture in the conduit 8 the control means 1 uses the values stored from steps 21-24 in the following relationship:

$$CH_4 = aP + bT + cT^2 + dThC(St) + eThC(R) + f \qquad (1)$$

Where:

$CH_4$       is the inferred methane percentage
P       is the absolute pressure of the fluid in the conduit
T       is the temperature of the fluid in the conduit
ThC(St)       is the thermal conductivity of the fluid in the conduit at a first temperature
ThC(R)       is the thermal conductivity of the fluid in the conduit at a second temperature and a, b, c, d, e and f are constants determined from experimental data using linear regression.

When pressure is measured in units of bar A, temperature is measured in degrees Celsius and thermal conductivity is measured in units of Watts/metre x Kelvin (W/m.K). The constants a - f have substantially the following values: a=-0.44, b= -0.39, c=0.0017, d=41, e = -42 and f=-181

Additionally or alternatively to step 25, at step 26 the ethane to methane ratio of the LNG mixture in the conduit 8 is determined using the values stored from steps 21-24 in the following relationship:

$$C2/C = gP + hT + iT^2 + jThC(St) + kThC(R) + l \qquad (2)$$

where:

C2/C is the inferred ethane/methane ratio as a percentage and
g, h, i, j, k and l are constants determined from experimental data using linear regression.

When pressure is measured in units of bar A, temperature is measured in degrees Celsius and thermal conductivity is measured in units of Watts/metre x Kelvin (W/m.K) the constants g - l have substantially the following values: g = 0.0053, h = 0.0051, i = -0.000024, j = -0.61, k = 0.66 and l = 3.14.

Either or both of the determined values of inferred methane percentage and inferred ethane/methane ratio may then be used by an engine management system. The engine management system could then ensure appropriate operation of an LNG fuelled engine despite fluctuations in the proportion of components of LNG supplied to the engine to achieve efficient operation. The engine management system could adjust the ignition timing in response to the LNG quality to stop the engine knocking. Alternatively or additionally the profile for the throttle control could be altered depending upon the quality of the LNG, eg if the LNG is poor the throttle would have to be opened more to obtain the same power. Alternatively either or both of the inferred methane percentage and inferred ethane/methane ratio may be used for any suitable purpose or displayed.

The apparatus for determining the proportion of at least one of the components of a fluid mixture was tested on four gas mixtures shown below chosen to represent the variation from "fresh" to "old" LNG.

| Test Gas | $CH_4$ | $C_2H_6$ | $C_3H_6$ | $nC_4$ |
|---|---|---|---|---|
| Sample 1 | 100.00 | 0.000 | 0.000 | 0.000 |
| Sample 2 | 94.996 | 4.240 | 0.472 | 0.292 |
| Sample 3 | 90.009 | 8.620 | 0.861 | 0.510 |
| Sample 4 | 85.006 | 12.700 | 1.430 | 0.864 |

Although this test was performed with gaseous mixtures, the test was found to work equally well with liquid mixtures as in LNG.

The test apparatus comprised a thermal conductivity sensor arranged to measure the thermal conductivity at two temperatures, an absolute pressure sensor and a temperature sensor all positioned in an environmental cabinet into which the test gas samples were sequentially introduced. Each of the sensors was calibrated against standard instruments. The temperature of each sample was varied by the environmental chamber and the absolute pressure inside the chamber was varied using a precision regulator.

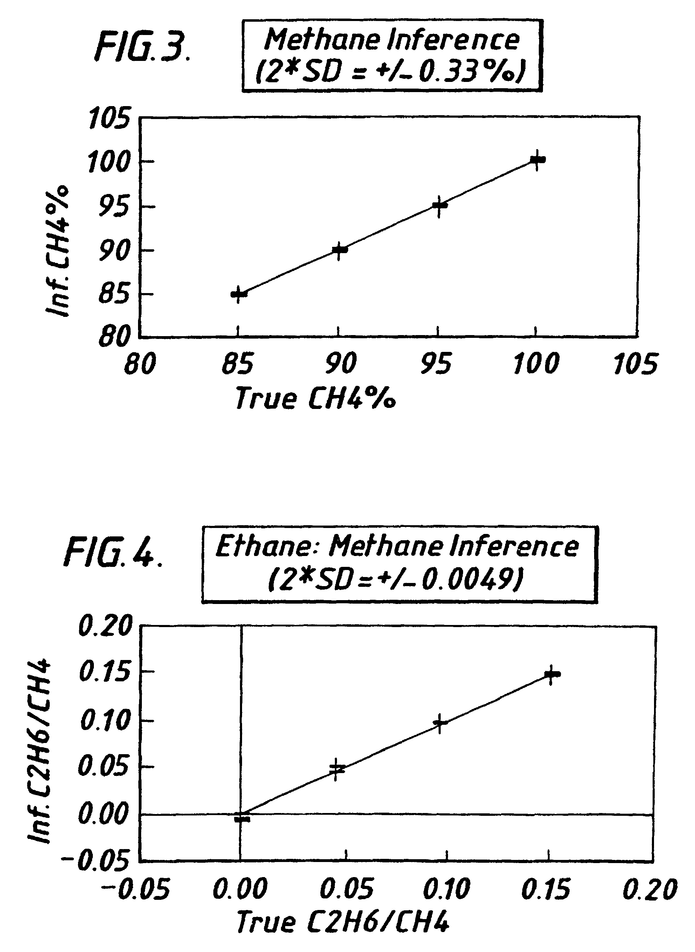

Figure 3 shows the determined or inferred methane percentage of each sample on the Y-axis and the actual methane percentage on the X-axis. The inferred values agree with the actual values very closely with only a very small error. Repeated experimental results using the above method were found to produce a range of two standard deviations within +/- 0.33% of the actual methane proportions. Thus using relationship No. 1 produced very precise data which would provide very efficient engine management.

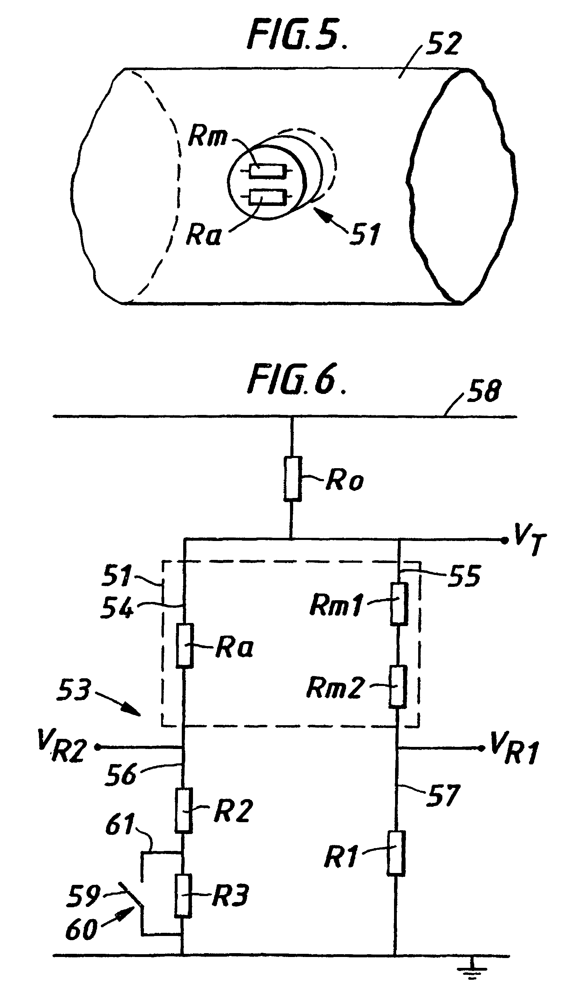

Figure 4 is similar to Figure 3 except showing the inferred ethane/methane ratio on the Y-axis and actual ethane/methane ratio on the X-axis. Repeated experimental results using the above method were found to produce a range of two standard deviations within +/- 0.0049% of the actual ethane/methane ratio which was even more precise than for the inferred methane proportion shown in Figure 3 using relationship No. 1. Again, using relationship No. 2, very precise data is produced which can be used for efficient engine management.

Experiments performed using the environmental chamber indicated that pressure variations caused only a minor difference to the final calculated proportions. This suggests that the absolute pressure sensor could be omitted for less precise requirements such as a cheaper sensor.

The thermal conductivity of the LNG can be measured by any suitable device or method. However, the preferred method uses a sensor having a resistor arranged to be surrounded by the fluid being tested. For precise measurements, the resistor needs to be thermally isolated from the substrate upon which it is supported so that heat generated by the resistor is substantially only transferred away from the resistor by conduction through the surrounding fluid. The resistor may be heated to a temperature above ambient by applying electrical power to the resistor to measure the thermal conductivity of the fluid at that temperature. The preferred sensor also has an additional resistor for measuring the ambient temperature. This resistor is thermally bonded to its substrate to ensure that it is maintained at ambient temperature. The effective resistances of the resistors are dependent upon their temperatures.

There is preferably provided a control circuit for a thermal conductivity sensor having a resistor arranged to be exposed to a fluid the thermal conductivity of which is to be determined, the control circuit comprising means to heat the resistor to at least two different temperatures and means arranged to provide a signal indicative of the thermal conductivity of the fluid at each of the at least two temperatures to which the resistor is heated. Such a control circuit is able to provide thermal conductivity measurements of a fluid at more than one temperature which is useful in the determination of the proportions of one or more of the constituents of LNG. Precise thermal conductivity measurements can be produced from such a sensor which is inexpensive, compact and robust. The control circuit may form part of the control means 1 described earlier.

Figure 5 diagrammatically shows a thermal conductivity sensor 51 as described above positioned in the wall of a gas pipe 52 with the thermal conductivity measuring resistor Rm and ambient temperature measuring resistor Ra exposed to the gas flowing inside the pipe. Of course the sensor 51 may be arranged to measure the thermal conductivity of any fluid whether it be flowing or static.

The resistance of these elements varies approximately with temperature according to the relationships:

$$(3) \qquad ....Rm = Rmo \, (1 + \alpha Tm)$$

$$(4) \qquad ....Ra = Rao \, (1 + \alpha \, Ta)$$

Where:-

Rmo    is the resistance of the thermal conductivity measuring resistor Rm at 0°C (Nominally 200Ω for two in series)

Ra0    is the resistance of the ambient temperature measuring resistor Ra at 0°C (Nominally 235Ω)

$\alpha$    is the temperature coefficient of resistance of the material of the resistor (nominally $5.5 \times 10^{-3}$/K).

Tm    is the temperature of the heated thermal conductivity measuring resistor.

Ta    is the temperature of the ambient temperature measuring resistor.

To enable the thermal conductivity of the gas surrounding the thermal conductivity measuring resistor Rm to be determined power must be applied to the resistor Rm by connecting it to a voltage source. From the temperature elevation and power applied the thermal conductivity of the gas surrounding the thermal conductivity measuring resistor Rm can be calculated using:-

$$(5) \qquad ....k = \Psi(P/\theta)$$

Where

k    is the thermal conductivity of the gas (Typically $3.65 \times 10^{-5}$ W/K for air at room temperature)
$\Psi$    is a scaling constant related to the construction of the sensor (order of 0.0036)
P    is the power dissipated in the heated measuring resistor Rm and
$\theta$    is the temperature of the heated measuring resistor Rm above ambient

The sensor 51 is shown in Figure 6 connected in a circuit 53 to control it so that thermal conductivity values for the gas flowing in the pipe 8 at a variety of temperatures can be determined.

The circuit 53 essentially consists of a bridge circuit having two arms 54, 55 connected to the supply voltage 58, in this case via current limiting resistor Ro and two arms 56, 57 connected to earth.

One of the arms 54 connected to the supply voltage 58 includes the ambient temperature measuring resistor Ra and the other arm *55* connected to the supply voltage 58 includes the thermal conductivity measuring resistor Rm, which in this example consists of two resistors Rm1, Rm2 connected in series.

The arm 56 connected between arm 54 and earth has in this example two resistors R2, R3 in series. R3 can be short circuited when desired. Resistor R3 can be short circuited by the appropriate control signal applied on line 59 to transistor 60 in path 61 parallel to resistor R3 to close the path 61 and by-pass resistor R3. The final arm 57 of the bridge has a resistor R1. Resistors R1, R2 and R3 enable the current through the heated thermal conductivity measuring resistor Rm and the ambient temperature measuring resistor Ra to be determined. They also limit the current passing through the sensor during fault conditions preventing or reducing sensor damage.

The bridge circuit maintains $V_{R1} = V_{R2}$ so that the bridge is "balanced' by adjusting the voltage $V_T$ at the top of the bridge. This controls the effective resistance of the heated measuring resistor Rm to be a constant multiple of the ambient temperature sensing resistor Ra given by the following relationship:

$$(6) \qquad .... Rm = Ra \ (R1/R2) \text{ when R3 is by-passed}$$

In the present example R1 = R2 (249Ω) so that

$$(7) \qquad .... Rm = Ra$$

This value of Rm corresponds to a temperature to which the thermal conductivity measuring resistor Rm is heated as determined by equation (3) which in the present example is approximately 80°C above ambient.

The thermal conductivity of the gas surrounding the thermal conductivity sensing resistor Rm at the temperature to which Rm is heated can then be determined using equation (5):

$$k = \Psi(P/\theta)$$

Since the temperature above ambient ($\theta$) is known as it is fixed by R1 and R2 and since $\Psi$ is a constant for a particular sensor, the thermal conductivity k at a particular temperature to which Rm is heated can be found by making a measure of the power (P) dissipated across Rm at that temperature.

The power P dissipated across Rm is given by:

$$(8) \qquad .... Power = I^2 Rm$$

Since the current passing through Rm is the same as that passing through R1, the current can be found by:

$$(9) \qquad .... I = V_{R1}/R1$$

Therefore the power dissipated across the thermal conductivity measuring resistor is found to be:

$$(10) \qquad .... power = V_{R1}^2 Rm/(R1)^2$$

substituting equation (10) into equation (5) using equations (3) and (4) gives the following result as explained in the appendix:

$$(11) \qquad .... k = \Psi V_{R1}^2 \ \alpha \ Rao \ Rmo/(Rao-Rmo)R1^2$$

As $\Psi$, $\alpha$, Rao, Rmo and R1 are all constants the thermal conductivity k of the gas surrounding the thermal conductivity sensing resistor Rm is proportional to the square of the voltage across R1:

$$(12) \qquad ..... k \propto V_{R1}^2$$

$$(13) \qquad .... k = z V_{R1}^2$$

The proportionality constant z will vary for each sensor manufactured due to resistor tolerances and so can be found by a separate calibration experiment using a gas of known thermal conductivity at the temperature to which Rm is heated. Hence the thermal conductivity of a gas surrounding resistor Rm at a first temperature can be determined directly from the square of the voltage across resistor R1 using a first predetermined proportionality constant z.

To determine the thermal conductivity of the gas at a second temperature, resistor R3 (20Ω) is included in series with R2. This is achieved by the application of an appropriate control signal on line 59 to transistor 60 to open path 61. In order to maintain $V_{R1} = V_{R2}$ in the bridge circuit the voltage $V_T$ is adjusted, changing the effective resistance of Rm which is given by:

$$(14) \qquad .... \; Rm = Ra(R1/(R2+R3))$$

This different effective value of Rm makes it operate at a different temperature from that when R3 is by-passed. The temperature is defined by equation (3). When R3 is in series with R2, Rm is heated to approximately 60°C above ambient.

The thermal conductivity of the gas surrounding resistor Rm at this second temperature can then be found from the square of the voltage across R1 as before using equation (13). However, in this case the proportionality constant z will be different and can be found by a separate calibration experiment using a gas of known thermal conductivity at the second temperature.

Hence by including or excluding R3 in arm 56 of the bridge circuit the temperature to which Rm is heated can be controlled to be one of two substantially predetermined values and the thermal conductivity of the surrounding gas can be determined at those two temperatures using predetermined constants. Of course more resistors can be controlled to be included or excluded in arm 6 of the bridge circuit to be able to determine the thermal conductivity of the surrounding gas at even more temperatures.

The temperatures to which the thermal conductivity measuring resistor Rm is heated are determined by the values of R1, R2 and R3 as shown by equations (3), (6) and (14). In the present example when R3 is by-passed, Rm is heated to approximately 80°C above ambient and when R3 is not by-passed Rm is heated to approximately 60°C above ambient.

However, a possible side effect of the circuit design is that the temperatures to which resistor Rm is heated and thus the temperatures at which the thermal conductivity is measured are dependent upon the ambient temperature which affects the resistance Ra in equations (6) and (14).

The ambient temperature could be measured with a thermometer but is more conveniently measured using the voltage $V_T$ at the top of the bridge circuit as shown in Figure 6. The resistance of the ambient temperature measuring resistor Ra can be determined assuming that the bridge circuit is balanced using ($V_{R1} = V_{R2}$) in either of the following equations:

$$(15) \qquad .... Ra = R2 \, (V_T - V_{R1})/V_{R1} \qquad \text{when R3 is by-passed}$$

$$(16) \qquad .... Ra = (R2 + R3) \, (V_T - V_{R1})/V_{R1} \qquad \text{when R3 is not by-passed}$$

The temperature T of ambient temperature sensing resistor Ra and thus the ambient temperature can then be found using equation (4).

Any variation in ambient temperature can thus be monitored and the corresponding adjustment of the temperature to which the thermal conductivity measuring resistor Rm is heated can be determined. A suitable proportionality constant z corresponding to the temperature to which Rm is heated can then be selected to ensure the provision of precise thermal conductivity measurements despite the variation in ambient temperature. The appropriate proportionality constant z is preferably looked up in a look-up table containing a proportionality constant z for each temperature at which the thermal conductivity measuring resistors Rm can be operated for the sensor being used.

Alternatively the values of the ratio of R1/R2 or R1/(R2 + R3) could be adjustable by at least one of R1 and R2 being variable to ensure that Rm is heated to a predetermined temperature. Alternatively additional resistors could be arranged to be able to be placed in series or parallel with at least one of R1 and R2 to adjust the ratio of R1/R2 or R1/(R2 + R3) to ensure that Rm is heated to a predetermined temperature.

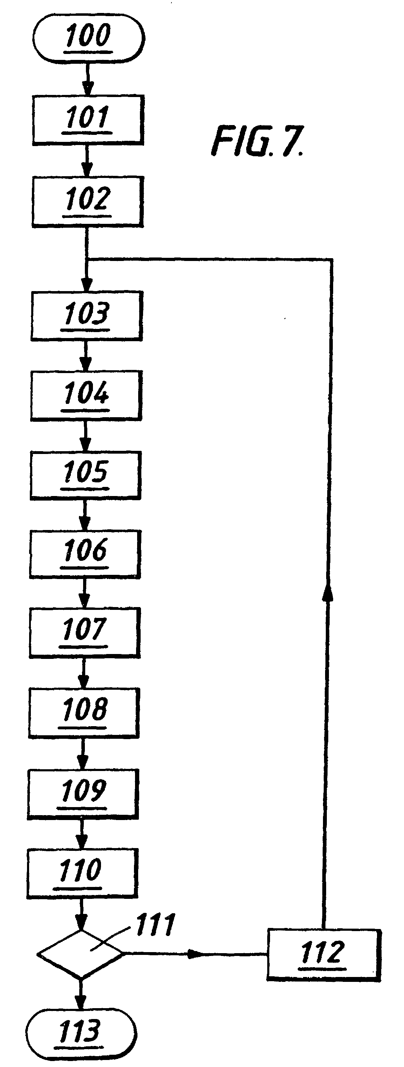

Figure 7 shows the sequence of operations for controlling a sensor to determine the thermal conductivity of a gas at two temperatures. The numbered steps have the following meaning:

100  Start
101  Control transistor 60 to by-pass resistor R3
102  Set counter to 0
103  Control $V_T$ such that $V_{R1} = V_{R2}$
104  Measure $V_{R1}$
105  Measure $V_T$
106  Determine Ra using equation 15 or 16 as appropriate.
107  Determine ambient temperature using equation (4) from which the temperature to which Rm is heated can be determined.
108  Select proportionality constant z eg from a look-up table for temperature to which Rm is heated.
109  Calculate thermal conductivity of gas from equation (13).

110 Increment counter

111 Counter = 2?

112 If no: Control transistor 60 to include resistor R3 and go to step 103.

113 If yes: Stop.

If desired step 108 could read:

"Adjust ratio of R1/R2 or R1/(R2 + R3) as appropriate". This adjustment could be achieved by any of the methods described earlier to heat thermal conductivity measuring resistor Rm to a predetermined temperature.

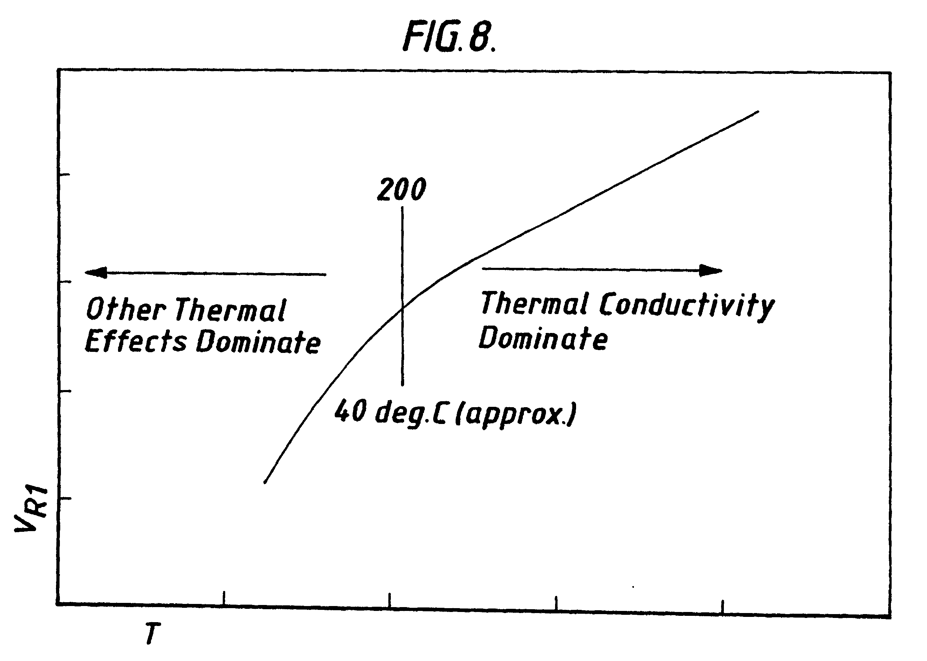

Figure 8 shows the output voltage $V_{R1}$ of the sensor 51 as a function of the temperature T of the thermal conductivity measuring resistor Rm for a fixed gas composition, ambient temperature and pressure. The sensor characteristic is found to follow an almost linear profile above a null point 200 whilst below the null point it is found to fall off sharply. From a comparison of experimental thermal conductivity results for a sample of fuel gas against theoretical calculated values, a good correlation resulted for values at temperatures above the null point and an increasingly poor correlation resulted below the null point. This may be due to thermal factors other than thermal conductivity such as convection and radiation increasingly dominating the performance of the sensor below the null point. However, above the null point even though these effects are still present, thermal conductivity dominates enabling precise thermal conductivity measurements to be achieved. For fuel gas the null point was found to be approximately 40°C. Thus thermal conductivity measuring resistor Rm is preferably operated above the null point 200 for a particular gas so that far more precise results are obtained.

To obtain the 80°C above ambient heating of Rm when R3 is by-passed in arm 56 of the bridge circuit, R1 and R2 are each selected to be 249Ω. To obtain the 60°C above ambient heating of Rm when R3 is in series with R2 in arm 56 of the bridge circuit, R3 is selected to be 20Ω.

It has been found that manufacturing variations in the values of the resistances of Rm and Ra in the sensor (generally worse than 5%) can cause the thermal conductivity measuring resistor Rm to operate at an unexpected or undesirable temperature. This could cause the thermal conductivity measuring resistor Rm to possibly operate below the null point and provide inaccurate thermal conductivity results.

To overcome the problems caused by the variability of the sensor resistances two additional electrical paths, each parallel to arm 56 of the bridge circuit between $V_{R2}$ and earth are preferably included, each path containing a resistor. Each path is selectively opened or closed preferably by the application of an appropriate control signal to a transistor in the path. Closing one electrical path whilst R3 is omitted from arm 56 of the bridge circuit introduces a resistor in parallel with resistor R2 of arm 56 elevating the temperature to which Rm is heated. Closing the other electrical path whilst R3 is included in arm 56 of the bridge circuit reduces the difference between the two temperatures to which Rm is elevated. The use of the additional paths prevents the heated temperature of Rm falling to undesirable levels which may be due to resistor tolerance of Rm and Ra. These additional electrical paths can also be used to ensure that Rm is heated to a predetermined temperature despite variations in ambient temperature as described earlier.

Many modifications of the above thermal conductivity sensor could be made. For example by the use of at least one of the controlled inclusion and exclusion of any number of resistors in series or the use of a variable resistor or the inclusion of resistors in parallel in an arm of the bridge circuit, the thermal conducivity measuring resistor Rm can be heated to a corresponding number of temperatures at which the thermal conductivity can be measured. Furthermore the control circuit 53 may include any suitable electrical elements instead of resistors.

**Appendix**

| | |
|---|---|
| Rm = | Rmo (1 + $\propto$ Tm) |
| and Tm = | Ta + θ |
| Rm = | Rmo (1 + $\propto$ Ta + $\propto$ θ) |
| Rm = | Rmo + Rmo $\propto$ Ta + Rmo $\propto$ θ |
| θ = | (Rm/Rmo$\propto$) - 1/$\propto$ - Ta |
| | = Rao ( + $\propto$ Ta)/Rmo $\propto$ - 1/$\propto$ - Ta |
| | = [Rao(1 + $\propto$ Ta) - Rmo - Rmo $\propto$ Ta]/Rmo$\propto$ |
| | = [Rao(1 + $\propto$Ta) - Rmo ( + $\propto$Ta)] / Rmo$\propto$ |
| θ = | (Rao - Rmo) (1 + $\propto$ Ta)/Rmo$\propto$ |
| k = | ΨP/θ |

$$k = \frac{\Psi V_{R1}^2 \; Rao \, (1 + \propto Ta) \propto Rmo}{R_1^2 \, (Rao - Rmo) \, (1 + \propto Ta)}$$

$$k = \Psi \, V_{R1}^2 \propto RaoRmo/R_1^2 \, (Rao-Rmo)$$

**Claims**

1. A method of determining the proportion of one of the components or the ratio of two of the components of a fluid mixture of liquid natural gas, the method comprising measuring the thermal conductivity of the liquid natural gas at a first temperature, measuring the thermal conductivity of the liquid natural gas at a second temperature and inferring the proportion of one of the components or the ratio of two of the components of the liquid natural gas from the two thermal conductivity measurements.

2. A method according to claim 1, including measuring the temperature of the liquid natural gas and inferring the proportion of at least one of the components or the ratio of two of the components of the liquid natural gas from the two thermal conductivity measurements and the temperature measurement.

3. A method according to claim 2, including measuring the absolute pressure of the liquid natural gas and inferring the proportion of at least one of the components or the ratio of two of the components of the liquid natural gas from the two thermal conductivity measurements, the temperature measurement and the absolute pressure measurement.

4. A method according to claim 3, wherein the proportion of one of the components or the ratio of two of the components of the liquid natural gas is inferred using the following expression:

$$X = uP + vT + wT^2 + xThC(St) + yThC(R) + z$$

where
X is inferred proportion of one of the components or the ratio of two of the components of the liquid natural gas;
P is the absolute pressure of a mixture;
T is the temperature of a mixture;
ThC(St) is the thermal conductivity of a mixture of a first temperature;
ThC(R) is the thermal conductivity of a mixture at a second temperature;
and u, v, w, x, y and z are constants.

5. A method according to claim 4, wherein
X is the inferred methane percentage;
P is measured in units of bar A;
T is measured in degrees Celsius
Thermal conductivity is measured in Watts/metre x Kelvin and the constants u - z have substantially the following values:
u = -0.44
v = -0.39
w = 0.0017
x = 41
y = -42 and
z = -181

6. A method according to claim 4, wherein
X is the inferred ethane/methane ratio;
P is measured in units of bar A;
T is measured in degrees Celsius;
Thermal conductivity is measured in Watts/metre x Kelvin and the constants u - z have substantially the following values:
u = 0.0053

v = 0.0051
w = -0.000024
x = -0.61
y = 0.66 and
z = 3.14

7. A liquid natural gas one component proportion or two component ratio determining apparatus, the apparatus comprising means (2) to measure the thermal conductivity of the liquid natural gas at two different temperatures and control means (1) to infer the proportion of one of the components or the ratio of two of the components of the liquid natural gas from the two thermal conductivity measurements.

8. An apparatus according to claim 7, including means (3) to measure the temperature of the liquid natural gas and the control means (1) being arranged to infer the proportion of one of the components or the ratio of two of the components of the liquid natural gas from the thermal conductivity measurements and the temperature measurement.

9. An apparatus according to claim 8, including means (4) to measure the absolute pressure of the liquid natural gas and the control means (1) being arranged to infer the proportion of one of the components or the ratio of two of the components of the liquid natural gas from the thermal conductivity measurements, the temperature measurement and the absolute pressure measurement.

10. An apparatus according to claim 9, wherein the control means (1) is arranged to infer the proportion of at least one of the components or the ratio of two of the components of the liquid natural gas using the following expression:

$$X = uP + vT + wT^2 + xThC(St) + yThC(R) + z$$

where
X is inferred proportion of one of the components or the ratio of two of the components of the liquid natural gas;
P is absolute pressure of a mixture;
T is the temperature of a mixture;
ThC(St) is the thermal conductivity of a mixture at a first temperature;
ThC(R) is the thermal conductivity of a mixture at a second temperature;
And u, v, w, x, y and z are constants.

11. An apparatus according to claim 10, wherein
X is the inferred methane percentage;
P is measured in units of bar A;
T is measured in degrees Celsius;
Thermal conductivity is measured in Watts/metre x Kelvin and the constants u - z have substantially the following values:
u = -0.44
v = -0.39
w = 0.0017
x = 41
y = -42 and
z = -181

12. An apparatus according to claim 10, wherein
X is the inferred ethane/methane ratio;
P is measured in units of bar A;
T is measured in degrees Celsius;
Thermal conductivity is measured in Watts/metre x Kelvin and the constants u - z have substantially the following values:
u = 0.0053
v= 0.0051
w = -0.000024
x = -0.61

y = 0.66 and
z= 3.14

13. An apparatus according to any of claims 7 to 12, wherein the means (2) to measure the thermal conductivity of the liquid natural gas at two different temperatures includes a resistor (Rm) arranged to be exposed to the liquid natural gas, means (53) to heat the resistor to two different substantially predetermined temperatures by application of suitable current or voltage to the resistor (Rm) and means (1) to determine the thermal conductivity of the mixture from an electrical characteristic of the resistor at each of the predetermined temperatures to which it is heated.

14. An engine management system for an LNG fuelled engine, the system including an apparatus according to any of claims 7 to 13, for determining the proportion of one of the components or the ratio of two of the components of LNG fuel and means to modify one or more engine parameters in response to the determined proportion of one of the components or the ratio of two of the components of the LNG fuel.

**Patentansprüche**

1. Verfahren zum Bestimmen des Anteils eines der Komponenten oder des Verhältnisses von zweien der Komponenten eines fluiden Gemisches aus flüssigem Erdgas, mit den Verfahrensschritten des Messens der Wärmeleitfähigkeit des flüssigen Erdgases bei einer ersten Temperatur, des Messens der Wärmeleitfähigkeit des flüssigen Erdgases bei einer zweiten Temperatur und des Folgerns der Proportion der einen der Komponenten oder des Verhältnisses von zweien der Komponenten des flüssigen Erdgases aus den beiden Wärmeleitfähigkeits-Messungen.

2. Verfahren nach Anspruch 1, einschließlich des Messens der Temperatur des flüssigen Erdgases und des Folgerns des Anteils mindestens der einen der Komponenten oder des Verhältnisses von zweien der Komponenten des flüssigen Erdgases aus den beiden Wärmeleitfähigkeits-Messungen und der Temperaturmessung.

3. Verfahren nach Anspruch 2, einschließlich des Messens des absoluten Drucks des flüssigen Erdgases und des Folgerns des Anteils mindestens der einen der Komponenten oder des Verhältnisses von zweien der Komponenten des flüssigen Erdgases aus den beiden Wärmeleitfähigkeits-Messungen, der Temperaturmessung und der Absolutdruck-Messung.

4. Verfahren nach Anspruch 3, bei dem der Anteil der einen der Komponenten oder das Verhältnis von zweien der Komponenten des flüssigen Erdgases unter Anwendung des folgenden Ausdrucks gefolgert bzw. abgeleitet wird:

$$X = uP + vT + wT^2 + xThC(St) + yThC(R) + z,$$

wobei
X der gefolgerte Anteil einer der Komponenten oder das Verhältnis von zweien der Komponenten des flüssigen Erdgases ist;
P der absolute Druck eines Gemisches ist;
T die Temperatur eines Gemisches ist;
ThC(St) die Wärmeleitfähigkeit eines Gemisches bei einer ersten Temperatur ist;
ThC(R) die Wärmeleitfähigkeit eines Gemisches bei einer zweiten Temperatur ist;
und u, v, w, x, y, und z Konstanten sind.

5. Verfahren nach Anspruch 4, bei dem
x der gefolgerte Methan-Prozentanteil ist;
P in Einheiten von bar A gemessen wird;
T in Grad Celsius gemessen wird;
Wärmeleitfähigkeit in Watt/Meter x Kelvin gemessen wird;
und die Konstanten u - z im wesentlichen folgende Werte haben:
u = -0,44
v = -0,39
w = 0,0017
x = 41

y = -42 und
z = -181.

6. Verfahren nach Anspruch 4, bei dem
X das Ethan/Methan-Verhältnis ist;
P in Einheiten von bar A gemessen wird;
T in Grad Celsius gemessen wird;
die Wärmeleitfähigkeit in Watt/Meter x Kelvin gemessen wird und
die Konstanten u - z im wesentlichen die folgenden Werte haben:
u = 0,0053
v = 0,0051
w = -0,000024
x = -0,61
y = 0,66 und
z = 3,14.

7. Vorrichtung zum Ermitteln des Anteils einer Komponente oder des Verhältnisses zweier Komponenten von flüssigem Erdgas mit Mitteln (2) zum Messen der Wärmeleitfähigkeit des flüssigen Erdgases bei zwei unterschiedlichen Temperaturen und mit einer Steuereinrichtung (1) zum Folgern des Anteils einer der Komponenten oder des Verhältnisses von zweien der Komponenten des flüssigen Erdgases aus den beiden Wärmeleitfähigkeits-Messungen.

8. Vorrichtung nach Anspruch 7 mit Mitteln (3) zum Messen der Temperatur des flüssigen Erdgases und mit der so ausgelegten Steuereinrichtung (1), daß sie den Anteil einer der Komponenten oder das Verhältnis von zweien der Komponenten des flüssigen Erdgases aus den Wärmeleitfähigkeits-Messungen und der Temperatur ableitet.

9. Vorrichtung nach Anspruch 8 mit Mitteln (4) zum Messen des absoluten Drucks des flüssigen Erdgases und mit der so ausgelegten Steuereinrichtung (1), daß sie den Anteil einer der Komponenten oder das Verhältnis von zweien der Komponenten des flüssigen Erdgases aus den Wärmeleitfähigkeits-Messungen, der Temperaturmessung und der Absolutdruck-Messung ableitet.

10. Vorrichtung nach Anspruch 9, bei der die Steuereinrichtung (1) so ausgelegt ist, daß sie den Anteil mindestens einer der Komponenten oder das Verhältnis von zweien der Komponenten des flüssigen Erdgases unter Anwendung des folgenden Ausdrucks ableitet:

$$X = uP + vT + wT^2 + xThC(St) + yThC(R) + z,$$

wobei
X der abgeleitete oder gefolgerte Anteil einer der Komponenten oder das Verhältnis von zweien der Komponenten des flüssigen Erdgases ist;
P der absolute Druck eines Gemisches ist;
T die Temperatur eines Gemisches ist;
ThC(St) die Wärmeleitfähigkeit eines Gemisches bei einer ersten Temperatur ist;
ThC(R) die Wärmeleitfähigkeit eines Gemisches bei einer zweiten Temperatur ist;
und u, v, w, x, y und z Konstanten sind.

11. Vorrichtung nach Anspruch 10, bei der
X der abgeleitete oder gefolgerte Methan-Prozentgehalt ist;
P in Einheiten von bar A gemessen ist;
T in Grad Celsius gemessen ist;
die Wärmeleitfähigkeit in Watt/Meter x Kelvin gemessen ist, und die Konstanten u - z im wesentlichen die folgenden Werte haben:
u = -0,44
v = -0,39
w = 0,0017
x = 41
y = -42 und

z = -181.

**12.** Vorrichtung nach Anspruch 10, bei der
X das abgeleitete oder gefolgerte Ethan/Methan-Verhältnis ist;
P in Einheiten von bar A gemessen ist;
T in Grad Celsius gemessen ist;
Wärmeleitfähigkeit in Watt/Meter x Kelvin gemessen ist, und die Konstanten u - z im wesentlichen die folgenden Werte haben:
u = 0,0053
v = 0,0051
w = -0,000024
x = -0,61
y = 0,66 und
z = 3,14.

**13.** Vorrichtung nach einem der Ansprüche 7 bis 12, bei der die Mittel (2) zum Messen der Wärmeleitfähigkeit des flüssigen Erdgases bei zwei unterschiedlichen Temperaturen einen Widerstand (Rm), der so angeordnet ist, daß er dem flüssigen Erdgas ausgesetzt ist, Mittel (53) zum Aufheizen des Widerstandes auf zwei unterschiedliche, im wesentlichen vorbestimmte Temperaturen durch Anlegen geeigneten Stromes oder geeigneter Spannung an den Widerstand (Rm), sowie Mittel (1) zum Bestimmen der Wärmeleitfähigkeit des Gemisches aus einer elektrischen Charakteristik des Widerstandes bei jeder der vorbestimmten Temperaturen, auf die er aufgeheizt wird, enthalten.

**14.** Triebwerk-Managementsystem für ein mit LNG betriebenes Triebwerk, mit einer Vorrichtung nach einem der Ansprüche 7 bis 13 zur Bestimmung des Anteils einer der Komponenten oder des Verhältnisses von zweien der Komponenten von LNG-Brennstoff sowie mit Mitteln zum Verändern eines oder mehrerer Parameter entsprechend dem festgestellten Anteil eines der Komponenten oder dem Verhältnis von zweien der Komponenten des LNG-Brennstoffs.

## Revendications

**1.** Procédé pour déterminer la proportion de l'un des composants ou le rapport de deux des composants d'un mélange fluidique de gaz naturel liquide, le procédé comprenant la mesure de la conductivité thermique du gaz naturel liquide à une première température, la mesure de la conductivité thermique du gaz naturel liquide à une deuxième température, et la déduction de la proportion de l'un des composants ou du rapport de deux des composants du gaz naturel liquide à partir des deux mesures de la conductivité thermique.

**2.** Procédé selon la revendication 1, comprenant la mesure de la température du gaz naturel liquide et la déduction de la proportion d'au moins un des composants ou du rapport de deux des composants du gaz naturel liquide à partir des deux mesures de la conductivité thermique et de la mesure de la température.

**3.** Procédé selon la revendication 2, comprenant la mesure de la pression absolue du gaz naturel liquide et la déduction de la proportion d'au moins un des composants ou du rapport de deux des composants du gaz naturel liquide à partir des deux mesures de la conductivité thermique, de la mesure de la température et de la mesure de la pression absolue.

**4.** Procédé selon la revendication 3, dans lequel la proportion de l'un des composants ou le rapport de deux des composants du gaz naturel liquide sont déduits en utilisant l'équation suivante :

$$X = uP + vT + wT^2 + xThC(St) + yThC(R) + z$$

où
X est la proportion déduite de l'un des composants ou le rapport de deux des composants du gaz naturel liquide ;
P est la pression absolue d'un mélange ;
T est la température d'un mélange ;

ThC(St) est la conductivité thermique d'un mélange à une première température ;
ThC(R) est la conductivité thermique d'un mélange à une deuxième température ;
et u, v, w, x, y et z sont des constantes.

5. Procédé selon la revendication 4, dans lequel
   X est le pourcentage de méthane déduit ;
   P est mesurée en unités de bar A ;
   T est mesurée en degrés Celsius ;
   La conductivité thermique est mesurée en Watts/mètre x Kelvin, et les constantes u - z ont substantiellement les valeurs suivantes :
   u = -0,44
   v = -0,39
   w = 0,0017
   x = 41
   y = -42 et
   z = -181

6. Procédé selon la revendication 4, dans lequel
   X est le rapport éthane/méthane déduit ;
   P est mesurée en unités de bar A ;
   T est mesurée en degrés Celsius ;
   La conductivité thermique est mesurée en Watts/mètre x Kelvin, et les constantes u - z ont substantiellement les valeurs suivantes :
   u = 0,0053
   v = 0,0051
   w = -0,000024
   x = -0,61
   y = 0,66 et
   z = 3,14

7. Appareil déterminant la proportion d'un composant ou le rapport de deux composants d'un gaz naturel liquide, l'appareil comprenant des moyens (2) pour mesurer la conductivité thermique du gaz naturel liquide à deux températures différentes, et des moyens de contrôle (1) pour déduire la proportion de l'un des composants ou le rapport de deux des composants du gaz naturel liquide à partir des deux mesures de la conductivité thermique.

8. Appareil selon la revendication 7, comprenant des moyens (3) pour mesurer la température du gaz naturel liquide, et les moyens de contrôle (1) étant agencés de manière à déduire la proportion de l'un des composants ou le rapport de deux des composants du gaz naturel liquide à partir des mesures de la conductivité thermique et de la mesure de la température.

9. Appareil selon la revendication 8, comprenant des moyens (4) pour mesurer la pression absolue du gaz naturel liquide, et les moyens de contrôle (1) étant agencés de manière à déduire la proportion de l'un des composants ou le rapport de deux des composants du gaz naturel liquide à partir des mesures de la conductivité thermique, de la mesure de la température et de la mesure de la pression absolue.

10. Appareil selon la revendication 9, dans lequel les moyens de contrôle (1) sont agencés de manière à déduire la proportion d'au moins un des composants ou le rapport de deux des composants du gaz naturel liquide en utilisant l'équation suivante :

$$X = uP + vT + wT^2 + xThC(St) + yThC(R) + z$$

où
X est la proportion déduite de l'un des composants ou le rapport de deux des composants du gaz naturel liquide
P est la pression absolue d'un mélange ;
T est la température d'un mélange ;
ThC(St) est la conductivité thermique d'un mélange à une première température ;

ThC(R) est la conductivité thermique d'un mélange à une deuxième température ;
et u, v, w, x, y et z sont des constantes.

11. Appareil selon la revendication 10, dans lequel
X est le pourcentage de méthane déduit ;
P est mesurée en unités de bar A ;
T est mesurée en degrés Celsius ;
La conductivité thermique est mesurée en Watts/mètre x Kelvin, et les constantes u - z ont substantiellement les valeurs suivantes :
u = -0,44
v = -0,39
w = 0,0017
x = 41
y = -42 et
z = -181

12. Appareil selon la revendication 10, dans lequel
X est le rapport éthane/méthane déduit ;
P est mesurée en unités de bar A ;
T est mesurée en degrés Celsius ;
La conductivité thermique est mesurée en Watts/mètre x Kelvin, et les constantes u - z ont substantiellement les valeurs suivantes :
u = 0,0053
v = 0,0051
w = -0,000024
x = -0,61
y = 0,66 et
z = 3,14

13. Appareil selon l'une quelconque des revendications 7 à 12, dans lequel les moyens (2) pour mesurer la conductivité thermique du gaz naturel liquide à deux températures différentes comprennent une résistance (Rm) agencée de manière à être exposée au gaz naturel liquide, des moyens (53) pour chauffer la résistance à deux températures prédéterminées substantiellement différentes en appliquant un courant ou une tension appropriés à la résistance (Rm), et des moyens (1) pour déterminer la conductivité thermique du mélange à partir d'une caractéristique électrique de la résistance à chacune des températures prédéterminées à laquelle elle est chauffée.

14. Système de commande de moteur pour un moteur au GNL, le système comprenant un appareil selon l'une quelconque des revendications 7 à 13, pour déterminer la proportion de l'un des composants ou le rapport de deux des composants du combustible GNL et des moyens pour modifier un ou plusieurs paramètres du moteur en réponse à une proportion déterminée de l'un des composants ou au rapport de deux des composants du combustible GNL.

# FIG.1.

FIG.2.

FIG.3. Methane Inference (2*SD = +/- 0.33%)

FIG.4. Ethane: Methane Inference (2*SD = +/- 0.0049)

## FIG.5.

## FIG.6.

FIG. 7.

FIG.8.

Thermal Conductivity Dominate

Other Thermal Effects Dominate

200

40 deg.C (approx.)

$V_{R1}$

T